## Europäisches Patentamt
## European Patent Office
## Office européen des brevets

(19)

(11) Veröffentlichungsnummer: **0 107 157**
**B1**

(12)

# EUROPÄISCHE PATENTSCHRIFT

(45) Veröffentlichungstag der Patentschrift:
**23.04.86**

(51) Int. Cl.⁴: **A 61 B  17/10**

(21) Anmeldenummer: **83110258.7**

(22) Anmeldetag: **14.10.83**

(54) Entklammerungszange.

(30) Priorität: **21.10.82  DE 3238898**

(43) Veröffentlichungstag der Anmeldung:
**02.05.84 Patentblatt 84/18**

(45) Bekanntmachung des Hinweises auf die Patenterteilung:
**23.04.86 Patentblatt 86/17**

(84) Benannte Vertragsstaaten:
**AT BE CH DE FR GB IT LI LU NL SE**

(56) Entgegenhaltungen:
**WO - A - 83/00428**
**CH - A - 186 088**
**US - A - 1 423 866**
**US - A - 4 026 520**

(73) Patentinhaber: **AESCULAP-WERKE AG vormals Jetter & Scheerer, Möhringer Strasse 125-146, D-7200 Tuttlingen (DE)**

(72) Erfinder: **Braun, Karl, Oberer Brühl 305, D-7201 Talheim (DE)**

(74) Vertreter: **Hoeger, Stellrecht & Partner, Uhlandstrasse 14c, D-7000 Stuttgart 1 (DE)**

## Beschreibung

Die Erfindung betrifft eine Entklammerungszange zum Aufbiegen von Hautklammern mit zwei verschwenkbar miteinander verbundenen Branchen, deren eine am freien Ende zwei parallele Zinken aufweist, die zwischen die Haut und den Steg der aufzubiegenden Klammer einziehbar sind, und mit einem Biegestempel am freien Ende der anderen Branche, der beim Zusammenschwenken der Branchen zwischen die beiden Zinken eintaucht, wobei eine der beiden Branchen die andere im Bereich der Schwenkverbindung U-förmig umgibt.

Entklammerungszangen dieser Art werden vorzugsweise aus Metall hergestellt und dienen dazu, Hautklammern, die Wundränder zum Verheilen zusammenhalten, nach dem Zusammenwachsen der Hautränder aufzubiegen und dadurch aus ihrer Position zu entfernen.

Normalerweise werden die beiden Branchen einer solchen Entklammerungszange mittels eines Lagerstiftes schwenkbar miteinander verbunden, beispielsweise mittels einer Schraube oder einer Niete. Eine derartige Entklammerungszange ist aus US-A-4 026 520 bekannt. Eine solche Ausgestaltung ist in der Herstellung aufwendig, da mindestens drei Teile miteinander verbunden werden müssen.

Es ist Aufgabe der Erfindung, eine Entklammerungszange der gattungsgemässen Art derart zu verbessern, dass ihre Herstellbarkeit vereinfacht wird, insbesondere mit dem Ziel, solche Entklammerungszangen als Wegwerfartikel zu konzipieren.

Diese Aufgabe wird bei einer Entklammerungszange der eingangs beschriebenen Art erfindungsgemäss dadurch gelöst, dass eine der beiden Branchen an ihren der anderen Branche zugewandten Seitenwänden in der Drehachse angeordnete Vorsprünge trägt, dass die andere Branche kreisförmige Vertiefungen zur Aufnahme der Vorsprünge aufweist, dass die Vorsprünge einen den Vertiefungen entsprechenden kreisförmigen Querschnitt haben, wobei zwei einander gegenüberliegende Seiten abgeflacht sind, und dass aus jeder kreisförmigen Vertiefung eine Nut zum Rand der anderen Branche läuft, deren Breite mindestens dem Abstand der abgeflachten Seiten der Vorsprünge entspricht, jedoch kleiner ist als der Durchmesser der kreisförmigen Vertiefung, und die so gerichtet ist, dass in der Betriebsstellung der beiden Branchen die kreisbogenförmigen Seitenteile der Vorsprünge zumindest teilweise an den zylindrischen Seitenwänden der Vertiefung anliegen.

Die Ausgestaltung macht es möglich, die beiden Branchen unmittelbar verschwenkbar miteinander zu verbinden, ohne dass dazu separate Teile, insbesondere Lagerstifte usw., notwendig sind. Es genügt nämlich, die beiden Branchen so zusammenzustecken, dass die abgeflachten Vorsprünge durch die Nuten in der anderen Branche in die Vertiefungen eintauchen, und die Branchen anschliessend in die Betriebsstellung zu verdrehen; dabei werden die Vorsprünge mit ihren kreisförmigen Bereichen in den im Querschnitt kreisförmigen Vorsprüngen exakt geführt.

Vorteilhaft ist es, wenn die Nut dieselbe Tiefe hat wie die Vertiefung, die Vertiefungen können jedoch auch durch eine einzige durchgehende Bohrung gebildet sein.

Vorzugsweise sind die Nut und die Abflachungen der Vorsprünge so orientiert, dass die Vorsprünge nur in die Nut einschiebbar sind, wenn die Branchen einen grösseren Öffnungswinkel einschliessen als er im Betrieb auftritt.

Bei einem bevorzugten Ausführungsbeispiel ist vorgesehen, dass beide Branchen an ihren einander zugewandten Seitenflächen Rastvorsprünge tragen, die beim Schliessen der Branchen aus der Zusammensetzstellung in die Betriebsstellung aneinander vorbeigleiten und ein Rückverschwenken aus der Betriebsstellung in die Zusammensetzstellung verhindern. Damit ist sichergestellt, dass die einmal zusammengesteckten Branchen unlösbar verbunden bleiben. Es genügt bei diesen Branchen, die eine Branche in Richtung der Nuten in der anderen Branche in diese einzuschieben und die beiden Branchen anschliessend zu schliessen, wobei die beiden Rastvorsprünge aneinander vorbeigleiten, bis die Entklammerungszange in der Betriebsstellung steht, d.h. in einer Stellung, die den Bereich zwischen der normalen Offenstellung und der Schliessstellung umfasst.

Dabei ist es vorteilhaft, wenn zumindest eine der beiden Rastvorsprünge eine schräge Gleitfläche für den anderen Rastvorsprung aufweist, die die beiden Seitenflächen der Branchen beim Schliessen in zunehmendem Masse voneinander entfernt. Dies erleichtert das Vorbeigleiten der Rastvorsprünge beim Verschwenken der beiden Branchen in die Betriebsstellung.

Es kann vorgesehen sein, dass der eine Rastvorsprung zapfenförmig und der andere linienförmig ausgebildet ist.

Bei einer weiteren bevorzugten Ausführungsform ist vorgesehen, dass an einer Branche am griffseitigen Ende auf der der anderen Branche zugewandten Seite zwei Führungsflächen angeordnet sind, die gegenüber der durch beide Branchen bestimmten Ebene geneigt sind und dass die andere Branche zwei komplementäre Führungsflächen trägt, die bei geschlossenen Branchen an den Führungsflächen der einen Branche anliegen. Diese Führungsflächen gewährleisten, dass die beiden Branchen beim Schliessen genau in der durch die beiden Branchen aufgespannten Instrumentenebene geführt werden, eine seitliche Verbiegung der Branchen beim Schliessen wird dadurch vermieden.

Besonders vorteilhaft ist es dabei, wenn die beiden Führungsflächen an jeder Branche in Branchenlängsrichtung gegeneinander versetzt sind, dadurch erhalten beide Führungsflächen bei gleicher Breite der Branchen eine grössere Länge, so dass die Führung über einen grösseren Winkelbereich gesichert ist.

Bei einer bevorzugten Ausführungsform ist vorgesehen, dass an dem Biegestempel ein nach unten ragender Vorsprung angeordnet ist, der den Steg der Klammer beim Schliessen der Branchen umgreift und gegen eine Anschlagfläche drückt, die sich an das branchenseitige Ende der Zinken anschliesst. Dies gewährleistet, dass die Klammer immer unmittelbar am branchenseitigen Ende der Zinken aufliegt, so dass auf die Zinken beim Verbiegen das kleinstmögliche Biegemoment ausgeübt wird.

Günstig ist es, wenn die Anschlagfläche senkrecht zur Längsrichtung der Zinken verläuft.

Vorzugsweise ist jeder der beiden Branchen als einstückiges Kunststoffteil ausgebildet.

Die nachfolgende Beschreibung bevorzugter Ausführungsformen der Erfindung dient im Zusammenhang mit der Zeichnung der näheren Erläuterung. Es zeigen:

Fig. 1 eine Seitenansicht einer Entklammerungszange;

Fig. 2 eine vergrösserte Teilseitenansicht des Biegebereichs der Entklammerungszange der Fig. 1;

Fig. 3 eine Schnittansicht längs Linie 3-3 in Fig. 1;

Fig. 4 eine Teillängsschnittansicht einer der beiden Branchen der Entklammerungszange der Fig. 1;

Fig. 5 eine Ansicht längs des Pfeiles A in Fig. 4;

Fig. 6 eine Teilseitenansicht der anderen Branche der Entklammerungszange der Fig. 1;

Fig. 7 eine Ansicht längs des Pfeiles B in Fig. 6;

Fig. 8 eine Querschnittsansicht durch Biegestempel und Zinken einer Entklammerungszange vor dem Aufbiegen einer Hautklammer;

Fig. 9 eine Ansicht ähnlich Fig. 8 nach dem Aufbiegen einer Hautklammer;

Fig. 10 eine Ansicht des vorderen Zangenbereichs vor dem Zusammenstecken der beiden Branchen;

Fig. 11 eine Schnittansicht längs Linie 11-11 in Fig. 10;

Fig. 12 eine Ansicht ähnlich Fig. 10 beim Einführen des Zapfens der einen Branche in die Vertiefung der anderen Branche;

Fig. 13 eine Schnittansicht längs Linie 13-13 in Fig. 12;

Fig. 14 eine Ansicht ähnlich Fig. 10 beim Aneinandergleiten der beiden Rastvorsprünge;

Fig. 15 eine Schnittansicht längs Linie 15-15 in Fig. 14;

Fig. 16 eine Ansicht ähnlich Fig. 10 der Zange in Betriebsstellung und

Fig. 17 eine Schnittansicht längs Linie 17-17 in Fig. 16.

Die in der Zeichnung dargestellte Entklammerungszange weist zwei Branchen 1 und 2 auf, die nach Art einer Zange oder Schere schwenkbar miteinander verbunden sind, und zwar nahe an einem Ende der beiden Branchen. Am gegenüberliegenden Ende der Branchen tragen diese in der Art von Scheren Fingeröffnungen 3 und 4. Ferner sind an den Innenseiten der beiden Branchen einander zugewandt an jeder Branche in Branchenlängsrichtung versetzt je zwei Führungsflächen 5 und 6 bzw. 7 und 8 angeformt, die alle gegenüber der durch die beiden Branchen aufgespannten Ebene schräg verlaufen, und zwar sind die gegeneinander versetzten Führungsflächen einer Branche jeweils entgegengesetzt geneigt. Die entsprechenden Führungsflächen an der anderen Branche weisen eine komplementäre Neigung zu den Führungsflächen der gegenüberliegenden Branche auf, so dass beim Schliessen der Branchen jeweils paarweise die Führungsflächen 5 und 7 und 6 und 8 aneinander zur Anlage gelangen und die beiden Branchen gegen eine Verschiebung aus der Branchenebene heraus sichern. Diese Sicherung macht es möglich, die verschwenkbare Lagerung der beiden Branchen aneinander weniger stabil auszugestalten; dies ist insbesondere dann von Vorteil, wenn die beiden Branchen, wie es vorzugsweise vorgesehen ist, jeweils einstückig aus Kunststoff gefertigt sind.

Die eine Branche 1 weist im Bereich der Schwenklagerung einen U-förmigen Querschnitt auf, d.h. im Lagerbereich weist diese Branche zwei parallele Seitenwände 9 und 10 auf, die über eine stegförmige Verdickung 11 der Branche 1 miteinander verbunden sind. Eine weitere Verbindung erfolgt in unmittelbarer Nähe des freien Endes der beiden Seitenwände über einen Steg 12.

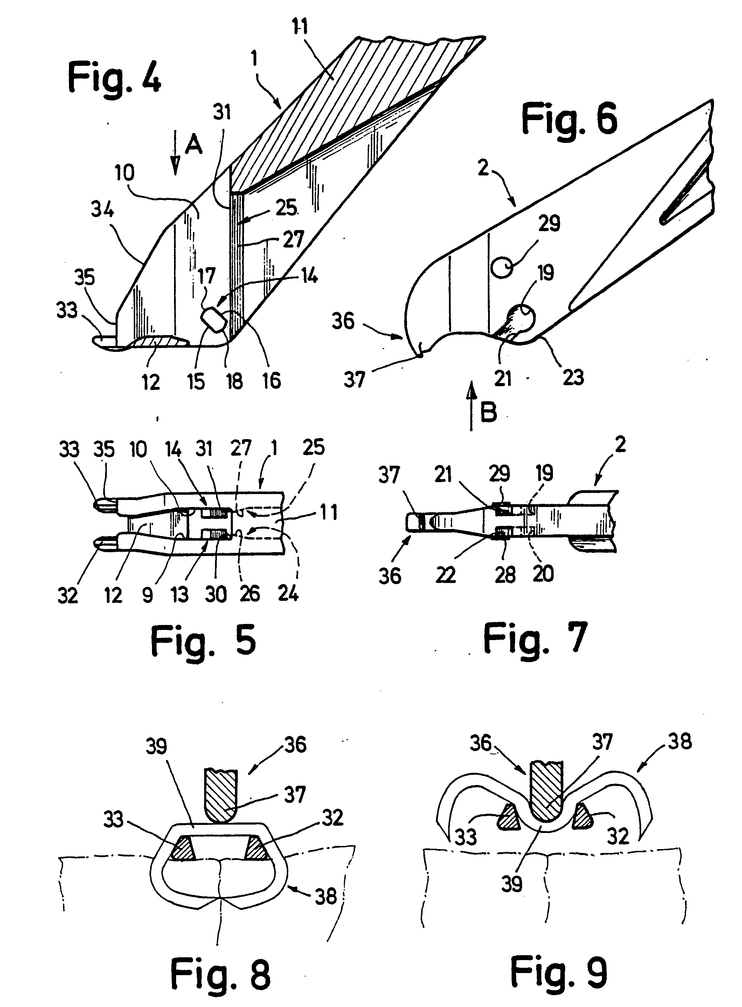

An beiden Seitenwänden 9 und 10 ist jeweils ein nach innen gerichteter Vorsprung 13 bzw. 14 angeformt, der einen im wesentlichen kreisförmigen Querschnitt aufweist, wobei jedoch die Vorsprünge auf einander gegenüberliegenden Seiten abgeflacht sind, so dass sich zwei parallele Seitenflächen 15 und 16 ergeben, die über kreiszylindrische Abschnitte 17 und 18 miteinander verbunden sind (Fig. 4). Die ebenen Seitenflächen 15 und 16 verlaufen dabei im wesentlichen senkrecht zur Branchenlängsrichtung.

Die andere Branche 2 hat im Lagerbereich eine Breite, die geringer ist als die lichte Weite der beiden Seitenwände 9 und 10 der Branche 1. Auf beiden Seiten weist diese Branche 2 Vertiefungen 19 bzw. 20 mit kreisförmigem Querschnitt auf, deren Durchmesser geringfügig grösser ist als der Durchmesser der kreisbogenförmigen Abschnitte 17 und 18 der Vorsprünge 13 bzw. 14. Von jeder Vertiefung 19 und 20 führt jeweils eine Nut 21 bzw. 22 zum Rand 23 der Branche 2, wobei die Breite beider Nuten geringfügig grösser ist als der Abstand der parallelen Seitenflächen 15 und 16 der Vorsprünge 13 bzw. 14. Die Breite der Nut ist jedoch kleiner als der Durchmesser der Vertiefungen 19 und 20. Die Richtung der Nut verläuft dabei im wesentlichen parallel zur Längsrichtung der Branche 2.

Die Nuten 21 und 22 können weniger tief sein als die Vertiefungen 19 und 20, vorzugsweise weisen jedoch Nuten und Vertiefungen dieselbe Tiefe auf. Die Vertiefungen können auch durch eine einzige durchgehende Bohrung gebildet sein.

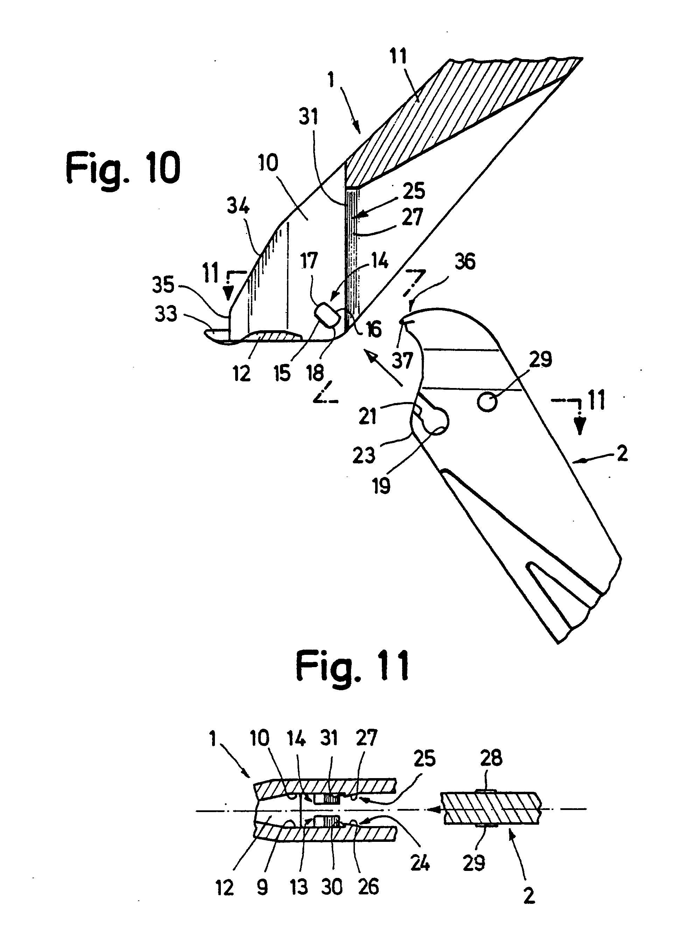

Jede der beiden Seitenwände 9 und 10 der Branche 1 weist ferner auf ihrer Innenseite einen balkenförmigen Rastvorsprung 24 bzw. 25 auf, der von der stegförmigen Verdickung 11 zum Rand der Branche 1 verläuft und im Querschnitt sägezahnförmig ausgebildet ist, wobei schräg verlaufende Gleitflächen 26 und 27 in Richtung zum freien Branchenende hin konvergieren (Fig. 11).

Die Branche 2 trägt im Verschwenkbereich auf gegenüberliegenden Seiten je einen zapfenförmigen Rastvorsprung 28 bzw. 29, der mit den Rastvorsprüngen 24 und 25 an der anderen Branche zusammenwirkt, wie sich aus der folgenden Beschreibung ergibt.

Beide Branchen werden vorzugsweise als einstückige Kunststoffteile hergestellt und können durch die beschriebene Ausgestaltung einfach durch Zusammenstecken schwenkbar miteinander verbunden werden. Die dazu erforderlichen Schritte sind in den Fig. 10 bis 17 dargestellt.

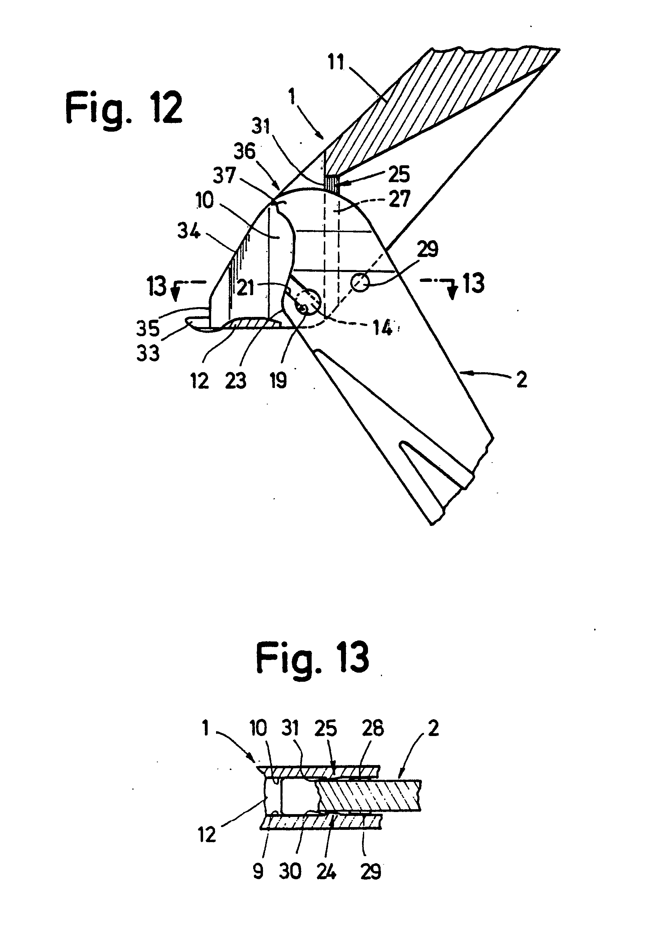

Zunächst wird die Branche 2 im wesentlichen senkrecht zur Branche 1 an diese herangeführt und mit ihrem freien Ende zwischen die beiden Seitenwände 9 und 10 eingeschoben, und zwar derart, dass die parallelen Seitenflächen 15 und 16 der Vorsprünge 13 und 14 in die Nuten 21 und 22 der Branche 2 eingeführt werden können (Fig. 10 und 11). Dabei treten die Vorsprünge 13 und 14 schliesslich in die Vertiefungen 19 und 20 ein, wobei sich in dieser Stellung die Rastvorsprünge 28 und 29 an der Branche 2 noch auf dem griffseitigen Ende der balkenförmigen Rastvorsprünge 24 und 25 befinden (Fig. 12 und 13).

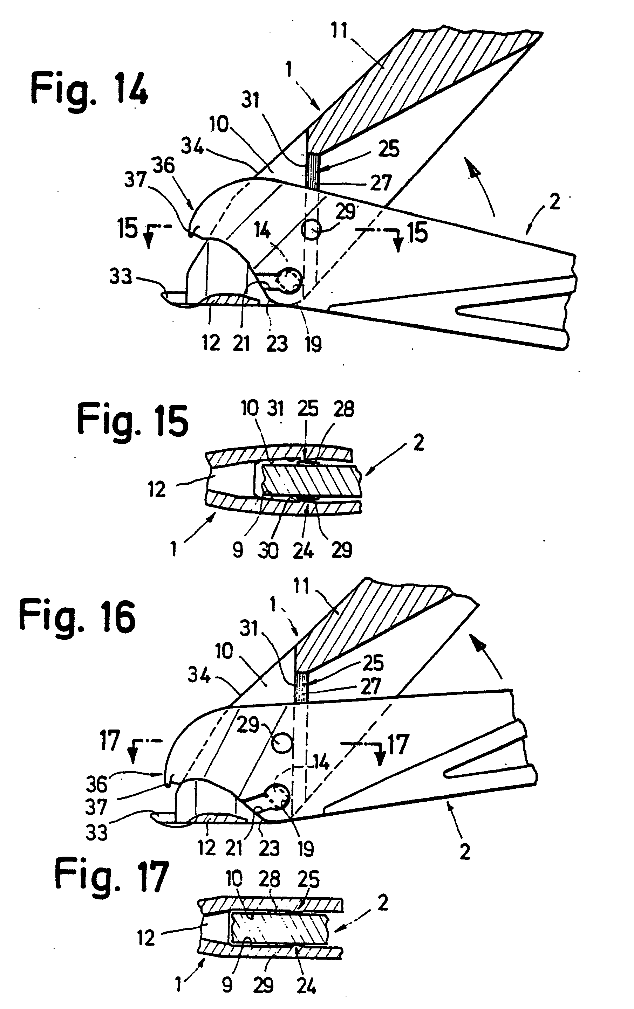

Beim Verschwenken der beiden Branchen in Schliessstellung verdrehen sich die Vorsprünge 13 und 14 in den Vertiefungen 19 und 20 derart, dass die Vorsprünge 13 und 14 mit ihren Seitenflächen 15 und 16 nicht mehr parallel zu den Nuten 21 und 22 stehen und somit auch nicht mehr durch gegenseitige Verschiebung der beidenBranchen aus der Vertiefung in die Nuten gelangen können. Gleichzeitig gleiten die Rastvorsprünge 28 und 29 längs der Gleitflächen 26 und 27 über die Rastvorsprünge 24 und 25 hinweg (Fig. 14 und 15), wobei sie die Seitenwände 9 und 10 elastisch auseinanderbiegen.

Sobald die Rastvorsprünge 28 und 29 an den Rastvorsprüngen 24 und 25 vorbeigeschwenkt sind, befindet sich die Entklammerungszange in der Betriebsstellung; eine Rückverschwenkung ist nicht möglich, da die Rastvorsprünge 28 und 29 dabei an senkrecht zu den Seitenwänden 9 und 10 verlaufenden Anschlagflächen 30 und 31 der Rastvorsprünge 24 und 25 stossen (Fig. 16 und 17). Die beiden Branchen sind nunmehr dauerhaft gegeneinander verschwenkbar miteinander verbunden.

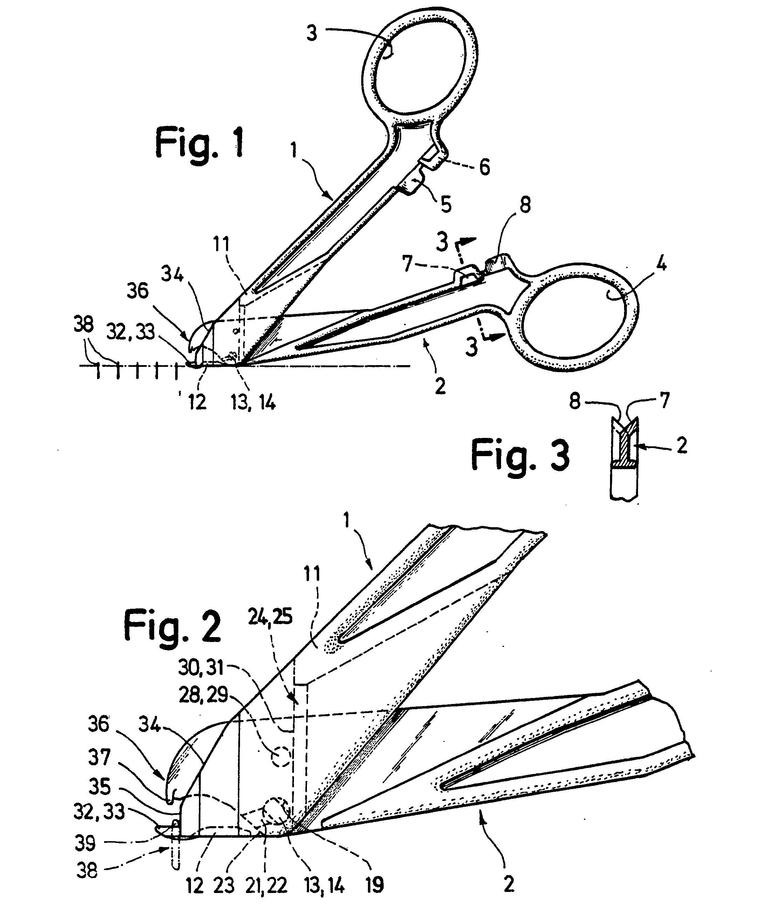

An ihrem freien Ende trägt jede Seitenwand 9 und 10 der Branche 1 jeweils eine Zinke 32 bzw. 33, die sich in wesentlichen parallel zum unteren Rand der Branche 1 erstrecken. Der obere Rand 34 der Branche 1 verläuft im Ansatzpunkt der beiden Zinken senkrecht zum unteren Rand und bildet dort eine senkrecht zur Zinkenlängsrichtung verlaufende Anschlagfläche 35 (Fig. 2).

Die Branche 2 ist an ihrem freien Ende als Biegestempel 36 ausgebildet und trägt einen nach unten ragenden Vorsprung 37, dessen Abstand von den Anschlagflächen 35 etwa der Drahtstärke der Hautklammer 38 entspricht (Fig. 2).

Zum Aufbiegen von Hautklammern wird die Entklammerungszange in der aus Fig. 1 ersichtlichen Weise mit den beiden Zinken unter eine Hautklammer 38 geschoben, wobei die beiden Zinken in der aus Fig. 8 ersichtlichen Weise an der Unterseite des Steges 39 einer Hautklammer 38 anliegen. Beim Schliessen der beiden Branchen drückt der Biegestempel 36 von oben auf den Steg 39 und biegt die Hautklammer in Offenstellung (Fig. 9). Dabei sorgt der Vorsprung 37 an der Unterseite des Biegestempels 36 dafür, dass der Steg 39 der Hautklammer 38 gegen die Anschlagfläche 35 gedrückt wird, d.h. die Hautklammer wird von den Zinken im Bereich ihres Ansatzpunktes unterstützt, so dass das von dem Biegestempel über den Steg 39 der Hautklammer 38 auf die Zinken ausgeübte Biegemoment in Folge der Verkürzung des Hebelarms der Zinken auf einen Minimalwert herabgesetzt wird.

Die vorstehend beschriebene Entklammerungszange besteht aus nur zwei Einzelteilen, die in einfachster Weise hergestellt werden können, insbesondere bei der Herstellung als Kunststoffteil. Beide Teile lassen sich in der beschriebenen Weise ohne Schwierigkeiten verschwenkbar miteinander verbinden, wobei diese Verbindung dauerhaft ausgestaltet werden kann. Diese vereinfachte Herstellung senkt die Herstellungskosten ganz erheblich, so dass die Entklammerungszange im Interesse einer sterilen Handhabung als Wegwerfteil ausgebildet werden kann.

## Patentansprüche

1. Entklammerungszange zum Aufbiegen von Hautklammern mit zwei verschwenkbar miteinander verbundenen Branchen, deren eine am freien Ende zwei parallele Zinken aufweist, die zwischen die Haut und den Steg der aufzubiegenden Klammer einschiebbar sind, und mit einem Biegestempel am freien Ende der anderen Branche, der beim Zusammenschwenken der Branchen zwischen die beiden Zinken eintaucht, wobei eine der beiden Branchen die andere im Bereich der Schwenkverbindung U-förmig umgibt, dadurch gekennzeichnet, dass eine der beiden Branchen (1 oder 2) an ihren der anderen Branche zugewandten Seitenwänden in der Drehachse angeordnete Vorsprünge (13, 14) trägt, dass die andere Branche (2 oder 1) kreisförmige Vertiefungen (19, 20) zur Aufnahme der Vorsprünge (13 bzw. 14) aufweist, dass die Vorsprünge (13, 14) einen den Vertiefungen (19 bzw. 20) entsprechenden kreisförmigen Querschnitt haben, wobei zwei einander gegenüberliegende Seitenflächen (15, 16) abgeflacht sind, und dass auf jeder kreisförmigen Vertiefung (19, 20) eine Nut (21 bzw. 22) zum Rand (23) der anderen Branche (2 oder 1) läuft, deren Breite mindestens dem Abstand der Seitenflächen (15, 16) der Vorsprünge (13 bzw. 14) entspricht, jedoch kleiner ist als der Durchmesser der kreisförmigen Vertiefung (19 bzw. 20), und die so gerichtet ist, dass in der Betriebsstellung der beidenBranchen (1, 2) kreisbogenförmige Abschnitte (17, 18) der Vorsprünge (13,14) zumindest teilweise an den zylindrischen Seitenwänden der Vertiefungen (19, 20) anliegen.

2. Zange nach Anspruch 1, dadurch gekennzeichnet, dass die Nuten (21, 22) dieselbe Tiefe haben wie die Vertiefungen (19 bzw. 20).

3. Zange nach Anspruch 1 oder 2, dadurch gekennzeichnet, dass die Nut (21, 22) und die Seitenflächen (15, 16) der Vorsprünge (13 bzw. 14) so orientiert sind, dass die Vorsprünge nur in die Nuten einschiebbar sind, wenn die Branchen (1, 2) einen grösseren Öffnungswinkel einschliessen als er im Betrieb auftritt.

4. Zange nach einem der voranstehenden Ansprüche, dadurch gekennzeichnet, dass beide Branchen (1, 2) an ihren einander zugewandten Seitenflächen Rastvorsprünge (24, 25 bzw. 28, 29) tragen, die beim Schliessen der Branchen (1 bzw. 2) aus der Zusammensetzstellung in die Betriebsstellung aneinander vorbeigleiten und ein Rückverschwenken aus der Betriebsstellung in die Zusammensetzstellung verhindern.

5. Zange nach Anspruch 4, dadurch gekennzeichnet, dass zumindest einer der beiden Rastvorsprünge (24, 25) eine schräge Gleitfläche (26 bzw. 27) für den anderen Rastvorsprung (28 bzw. 29) aufweist, die die beiden Seitenflächen der Branchen (1, 2) beim Schliessen in zunehmendem Masse voneinander entfernt.

6. Zange nach einem der Ansprüche 4 oder 5, dadurch gekennzeichnet, dass der eine Rastvorsprung (28, 29) zapfenförmig und der andere linienförmig ausgebildet ist.

7. Zange nach einem der voranstehenden Ansprüche, dadurch gekennzeichnet, dass an einer Branche (1) am griffseitigen Ende auf der der anderen Branche zugewandten Seite zwei Führungsflächen (5, 6) angeordnet sind, die gegenüber der durch beide Branchen (1, 2) bestimmten Ebene geneigt sind und dass die andere Branche zwei komplementäre Führungsflächen (7, 8) trägt, die bei geschlossenen Branchen (1, 2) an den Führungsflächen (5, 6) der einen Branche anliegen.

8. Zange nach Anspruch 7, dadurch gekennzeichnet, dass die beiden Führungsflächen (5, 6 bzw. 7, 8) an jeder Branche (1 bzw. 2) in Branchenlängsrichtung gegeneinander versetzt sind.

9. Zange nach einem der voranstehenden Ansprüche, dadurch gekennzeichnet, dass an dem Biegestempel (36) ein nach unten ragender Vorsprung (37) angeordnet ist, der den Steg (39) der Klammer (38) beim Schliessen der Branchen (1, 2) umgreift und gegen eine Anschlagfläche (35) drückt, die sich an das branchenseitige Ende der Zinken (32, 33) anschliesst.

10. Zange nach Anspruch 9, dadurch gekennzeichnet, dass die Anschlagfläche (35) senkrecht zur Längsrichtung der Zinken (32, 33) verläuft.

11. Zange nach einem der voranstehenden Ansprüche, dadurch gekennzeichnet, dass jede der beiden Branchen (1, 2) als einstückiges Kunststoffteil ausgebildet ist.

## Claims

1. Unclamping tongs for bending open skin staples, having two pivotably connected arms, one arm having at its free end two parallel prongs, which can be inserted between the skin and the crosspiece of the staple which is to be bent open, and the other arm having at its free end a bending punch, which plunges between the two prongs, one of the two arms surrounding the other in a U-shape in the region of the pivot connection, characterised in that, on its lateral walls facing the other arm, one of the two arms (1 or 2) has projections (13, 14) arranged in the pivot axis, the other arm (1 or 2) comprises circular recesses (19, 20) for receiving the projections (13 or 14), the projections (13, 14) have a circular cross-section corresponding to the recesses (19 or 20), two oppositely disposed lateral surfaces (15, 16) being bevelled, and a groove (21 or 22) extends out of each circular recess (19, 20) as far as the edge (23) of the other arm (2 or 1), the width of the groove corresponding at least to the distance between the lateral surfaces (15, 16) of the projections (13 or 14), but being smaller than the diameter of the circular recess (19 or 20) and being directed such that, when the two arms (1, 2) are in the operating position, arcuate sections (17, 18) of the projections (13, 14) rest at least partially against the cylindrical lateral surfaces of the recesses (19, 20).

2. Tongs according to claim 1, characterised in that the grooves (21, 22) have the same depth as the recesses (19 or 20).

3. Tongs according to claim 1 or 2, characterised in that the groove (21, 22) and the lateral surfaces (15, 16) of the projections (13 or 14) are positioned such that the projections can only be inserted into the grooves if the arms (1, 2) form a larger aperture angle than that which exists during operation.

4. Tongs according to any one of the above claims, characterised in that, on their oppositely disposed lateral surfaces, both arms (1, 2) comprise locking projections (24, 25 or 28, 29), which slide past one another when the arms (1 or 2) are closed from the assembly position into the operating position and which prevent a pivoting back from the operating position into the assembly position.

5. Tongs according to claim 4, characterised in that at least one of the two locking projections (24, 25) comprises an inclined sliding surface (26 or 27) for the other locking projection (28 or 29), which sliding surface separates the two lateral surfaces of the arms (1, 2) from one another to an increasing degree during closure.

6. Tongs according to any one of claims 4 or 5, characterised in that one locking projection (28, 29) is conical and the other is linear.

7. Tongs according to any one of the above claims, characterised in that on one arm (1) two guide surfaces (5, 6) are arranged at the gripping end on the side facing the other arm, which guide surfaces are inclined with respect to the plane defined by the two arms (1, 2), and the other arm has two complementary guide surfaces (7, 8), which rest against the guide surfaces (5,6) of the first arm when the arms (1, 2) are closed.

8. Tongs according to claim 7, characterised in that the two guide surfaces (5, 6 or 7, 8) in each arm (1 or 2) are offset with respect to one another in the longitudinal direction of the arms.

9. Tongs according to any one of the preceding claims, characterised in that a downward projection (37) is arranged on the bending punch (36), which projection embraces the crosspiece (39) of the staple (38) when the arms are closed and presses against an abutment surface (35), which is attached to the end of the prongs (32, 33) facing the arms.

10. Tongs according to claim 9, characterised in that the abutment surface (35) extends vertical to the longitudinal direction of the prongs (32, 33).

11. Tongs according to any one of the preceding claims, characterised in that each of the two arms (1, 2) is formed as a one-piece plastics material part.

## Revendications

1. Dégrafeuse pour dégrafer des agrafes pour la peau, comportant deux branches, reliées l'une à l'autre avec possibilité de pivotement, dont l'une présente à son extrémité libre deux dents parallèles qui peuvent se glisser entre la peau et l'âme de l'agrafe à dégrafer, et présentant à l'extrémité libre de l'autre branche un poinçon de pliage qui, lorsque les branches pivotent, pénètre entre les deux dents, étant précisé que l'une des deux branches entoure l'autre en forme de U au voisinage de la liaison pivotante, caractérisée en ce que l'une des deux branches (1 ou 2) porte sur ses parois latérales orientées vers l'autre branche, des saillies (13, 14) disposées dans l'axe de rotation; en ce que l'autre branche (2 ou 1) présente des logements de forme circulaire (19, 20) pour recevoir les saillies (13 et 14) en ce que les saillies (13, 14) ont une section de forme circulaire correspondant aux logements (19 et 20) étant précisé que sur deux surfaces latérales (15, 16) situées en face l'une de l'autre sont prévus des méplats; et en ce que, depuis chaque logement de forme circulaire (19, 20) part, vers le bord (23) de l'autre branche (2 ou 1) une rainure (21 et 22) dont la largeur correspond au moins à la distance des surfaces latérales (15, 16) des saillies (13 et 14), mais est inférieure au diamètre du logement de forme circulaire (19 et 20) et qui est dirigée de façon qu'en position d'utilisation des deux branches (1, 2) des portions en forme d'arc de cercle (17, 18) des saillies (13, 14) s'appuient au moins partiellement contre les parois latérales cylindriques des logements (19, 20).

2. Dégrafeuse selon la revendication 1, caractérisée en ce que les rainures (21, 22) ont la même profondeur que les logements (19, 20).

3. Dégrafeuse selon la revendication 1 ou la revendication 2, caractérisée en ce que la rainure (21, 22) et les surfaces latérales (15, 16) des saillies (13 et 14) sont orientées de façon que les saillies ne puissent glisser dans les rainures que lorsque les branches (1, 2) font entre elles un angle d'ouverture supérieur à celui qui apparaît en cours d'utilisation.

4. Dégrafeuse selon l'une des revendications précédentes, caractérisée en ce que les deux branches (1, 2) portent, sur leurs surfaces latérales tournées l'une vers l'autre, des saillies de crantage (24, 25 et 28, 29) qui, lorsque l'on ferme les branches (1 et 2) pour passer de la position d'assemblage à la position d'utilisation, glissent par delà l'une l'autre et interdisent un pivotement en arrière pour passer de la position d'utilisation à la position d'assemblage.

5. Dégrafeuse selon la revendication 4, caractérisée en ce qu'au moins l'une des deux saillies de crantage (24, 25) présente, pour l'autre saillie de crantage (28 ou 29) une surface de glissement oblique (26 ou 27) qui, lors de la fermeture, éloigne l'une de l'autre dans une mesure croissante les deux surfaces latérales des branches (1, 2).

6. Dégrafeuse selon l'une des revendications 4 ou 5, caractérisée en ce que l'une des saillies de crantage (28, 29) a la forme d'un téton et l'autre une forme linéaire.

7. Dégrafeuse selon l'une des revendications précédentes, caractérisée en ce que sur l'une des branches (1), à l'extrémité côté prise, sont disposées, sur la face tournée vers l'autre branche, deux surfaces de guidage (5, 6) qui sont inclinées par rapport au plan défini par les deux branches (1, 2); et en ce que l'autre branche porte deux surfaces de guidage complémentaires (7, 8) qui lorsque les branches (1, 2) sont fermées s'appuient contre les surfaces de guidage (5, 6) de la première branche.

8. Dégrafeuse selon la revendication 7, caractérisée en ce que les deux surfaces de guidage (5, 6 et 7, 8) sont décalées l'une par rapport à l'autre sur chaque branche (1 et 2) dans la direction longitudinale de la branche.

9. Dégrafeuse selon l'une des revendications précédentes, caractérisée en ce que sur le poinçon de pliage (36) est disposée une saillie (37) dépassant vers le bas, qui enserre l'âme (39) de l'agrafe (38) lors de la fermeture des branches (1, 2) et l'appuie contre une surface de butée (35) qui se raccorde à l'extrémité des dents (32, 33) côté branche.

10. Dégrafeuse selon la revendication 9, caractérisée en ce que la surface de butée (35) court perpendiculairement à la direction longitudinale des dents (32, 33).

11. Dégrafeuse selon l'une des revendications précédentes, caractérisée en ce que chacune des deux branches (1,2) est conçue comme élément plastique d'une seule pièce.

0 107 157

Fig. 1

Fig. 3

Fig. 2

7

Fig. 4

Fig. 6

Fig. 5

Fig. 7

Fig. 8

Fig. 9

# Fig. 10

# Fig. 11

# Fig. 12

11
1
31
36
37
10
34
25
27
13
29
13
21
35
14
33
12  23  19
2

# Fig. 13

1
10  31  25  28
2
12
9  30  24  29

Fig. 14

Fig. 15

Fig. 16

Fig. 17